Europäisches Patentamt

European Patent Office

Office européen des brevets

(19)

(11) Publication number: **0 076 696**
**A2**

(12) **EUROPEAN PATENT APPLICATION**

(21) Application number: **82305295.6**

(22) Date of filing: **05.10.82**

(51) Int. Cl.³: **C 12 N 9/16**
**C 12 P 19/34**
**//C12R1/13**

(30) Priority: **05.10.81 JP 157448/81**

(43) Date of publication of application:
**13.04.83 Bulletin 83/15**

(84) Designated Contracting States:
**CH DE FR GB IT LI**

(71) Applicant: **KABUSHIKI KAISHA YAKULT HONSHA**
**1-19, Higashishinbashi 1-chome**
**Minato-ku Tokyo 105(JP)**

(72) Inventor: **Khosaka, Toshiyuki**
**Yakult Central Inst. for Microbiological Research**
**1796 Yaho Kunitachi Tokyo(JP)**

(72) Inventor: **Sakurai, Toshizo**
**7-19, 2-chome Nakahara Mitaka**
**Tokyo(JP)**

(72) Inventor: **Mutai, Masahiko**
**988, 4-chome Shimizu Higashiyamato**
**Tokyo(JP)**

(74) Representative: **Sanderson, Laurence Andrew et al,**
**Sanderson & Co. 97 High Street**
**Colchester Essex C01 1TH(GB)**

(54) A restriction enzyme and a method for production thereof.

(57) A restriction enzyme having a specificity such that it can recognize a palindromic, hexanucleotide sequence 5'-GGCGCC-3', and can cleave the recognition sequence 5'-GGCGCC-3' between the two C's at the 3' ends, so as thus to generate DNA fragments with tetranucleotide long 3'-protruding ends, GCGC-3'. A method for production of this restriction enzyme employs strains belonging to *Bifidobacterium breve*, e.g. YIT 4006 having the Deposition No. FERM-P3906.

./...

EP 0 076 696 A2

Fig. 4

## A RESTRICTION ENZYME AND A METHOD

## FOR PRODUCTION THEREOF

The present invention relates to a restriction enzyme and a method for production thereof. More specifically, the present invention relates to a site specific endonuclease which is capable of recognizing a specific nucleotide sequence contained in a deoxyribonucleic acid (hereinafter referred to as a DNA) and of cleaving the DNA at a specific site which is located inside or close to the foregoing nucleotide sequence that the restriction enzyme successfully recognizes, and relates to a method for production of the foregoing restriction enzyme out of a productive strain which is easy to grow and handle, has a large yield for production of the foregoing restriction enzyme with less possibility to produce contaminating enzyme activities, and readily maintains the inherent enzyme activity for a long time without requiring a severe control of environmental conditions.

An endonuclease is defined as an enzyme which is capable of cleaving a DNA, and a restriction enzyme is defined as a kind of endonuclease which is capable of recognizing a specific nucleotide sequence contained in a DNA and of cleaving the DNA at a specific site which is located inside or close to the nucleotide sequence recognized by the restriction enzyme. Endonucleases particularly restriction enzymes are useful for physical mapping of a microorganism particularly of a DNA, analysis of nucleotide sequences contained in a DNA, and/or isolation of genes from a microorganism. Therefore, restriction enzymes have become an indispensable tool for recombinant DNA techniques.

In the prior art, we know fairly limited kinds of restriction enzymes. Incidentally, the kinds or types of nucleotide sequences which can be recognized by a restriction enzyme are fairly limited. It is quite natural that if a wider variety of restriction enzymes and of nucleotide . sequences can be recognized by a restriction enzyme that will be useful for recombinant DNA techniques. Further, a variety of sites at which a nucleotide sequence is cleaved is useful for recombinant DNA techniques. In summary, diversification of specificity in the enzyme activity is one of the major parameters to which development efforts are extensively directed.

In the prior art, it is conventional that a restriction enzyme is isolated from bacteria. It is of course advantageous, if a type of bacteria which satisfies the following requirements:

(1) Nonpathogenic,

(2) Easy to cultivate, and

(3) Capable of producing a large yield of a useful restriction enzyme accompanied by a small yield of contaminating enzyme activities,

can be employed as a productive strain.

It might be  assumed that some of the intestinal bacteria would satisfy the foregoing requirements, and some of the intestinal bacteria have been assumed to be potential productive strains for production of restriction enzymes. From the theoretical viewpoint  some of the bacteria belonging to Bifidobacterium should be included in the foregoing category, because they are easy to cultivate on a large scale and they are  inclined to be accompanied by a large yield, whenever they are capable of producing some type of endonuclease.  Exemplary strains which have been found to be employable as productive strains are itemized below:

|                   | Strain |              |
| Genus & Species   | Name   | Deposition No. |
|-------------------|--------|----------------|
| B. breve          | YIT4006 | FERM-P3906    |
| B. Bifidum        | YIT4007 | FERM-P5871    |
| B. infantis       | 659     | ATCC25962     |
| B. infantis       | S76e    | ATCC15702     |
| B. longum         | E194b   | ATCC15707     |
| B. thermophilum   | RU326   | ATCC25866     |
| B. breve          | S1      | ATCC15700     |
| B. breve          | S50     | ATCC15698     |

An object of the present invention is to provide a restriction enzyme which has a novel specificity as regards the recognition sequence, defined as a nucleotide sequence which can be specifically recognized by a specific restriction enzyme, and as regards the cleavage site, defined as a site which is located in the nucleotide sequence recognized by the specific restriction enzyme and at which the DNA is cleaved. More specifically, an object of the present invention is to provide a restriction enzyme which has an enzyme activity (i) with which the enzyme recognizes a palindromic hexanucleotide sequence,

```
5'-GGCGCC-3'
3'-CCGCGG-5',
```

in double stranded DNA's, and (ii) with which the double stranded DNA's are cleaved between the two C's at the 3' ends, so that DNA fragments with tetranucleotide long 3'-protruding ends, GCGC-3', are generated.

Another object of the present invention is to provide a method for production of the foregoing restriction enzyme employing bacteria which are nonpathogenic, easy to cultivate on a large scale, and capable of producing a large yield of the restriction enzyme not accompanied by any significant yield of contaminating enzyme activities. More specifically, another object of the present invention is to provide a method for production of the foregoing restriction enzyme employing one or more of the bacteria belonging to Bifidobacterium breve as the productive strain.

According to a first aspect of the present invention, there is provided a restriction enzyme having a specifity, such that it can recognize a palindromic hexanucleotide sequence

```
5'-GGCGCC-3'
3'-CCGCGG-5',
```

in double stranded DNA's and can cleave the phosphodiester
bonds contained in the double stranded DNA's between the
two C's at the 3' ends shown by arrows on the following
sequence

$$\downarrow$$
5'-GGCGCC-3'
3'-CCGCGG-5',
$$\uparrow$$

so that deoxyribonucleic acid fragments having tetranucleotide
long 3'-protruding ends are generated, and also having
an optimum pH value of 7.4 within by a stable range
thereof of 6.5 through 8.3, and
an optimum temperature range of 35 through 39°C.

One embodiment of a restriction enzyme in accordance
with the first aspect of the present invention has such a specificity that (i) the restriction enzyme is capable of
recognizing a palindromic hexanucleotide sequence,

5'-GGCGCC-3'
3'-CCGCGG-5',

in double stranded DNA's, and (ii) the restriction enzyme
is capable of cleaving the phosphodiester bonds contained
in the double stranded DNA's between the two C's at the 3'
ends shown by arrows on the following sequence,

$$\downarrow$$
5'-GGCGCC-3'
3'-CCGCGG-5',
$$\uparrow$$

so that the restriction enzyme is capable of generating DNA
fragments with tetranucleotide long 3'-protruding ends,
an optimum pH value of 7.4 within by the stable range
of 6.5-8.3, an optimum temperature range of 35-39°C, the
ability to be activated by 1-15 mM $Mg^{2+}$ ions without

assistance by a cofactor such as ATP and/or S-adenosyl-methionine, and the ability to have its enzyme activity inhibited by NaCl.

According to a second aspect of the present invention, there is provided a method for production of a restriction enzyme comprising the steps of:

cultivating a strain belonging to Bifidobacterium breve,

harvesting cells of said strain,

breaking said cells,

separating a crude extract of said cells,

fractionating said crude extract, and

purifying said crude extract, at least once so as thus to isolate therefrom a restriction enzyme having specifity for recognizing a palindromic hexanucleotide sequence,

$$5'\text{-GGCGCC-}3'$$
$$3'\text{-CCGCGG-}5',$$

in double stranded DNA's, and for cleaving the deoxyribonucleic acids between the two C's at the 3' ends.

A preferred productive strain for use in the foregoing second aspect of the present invention is a specific strain of which the Deposition No. is FERM-P3906.

One embodiment of a method for production of a restriction enzyme in accordance with the second aspect of the present invention includes the steps of cultivating a productive strain belonging to Bifidobacterium breve, e.g.

YIT 4006 having its Deposition No. FERM-P3906, anaerobically in a medium; harvesting the cells thereof; breaking the cells e.g. by sonification applied after lysozyme treatment; separating the crude extract thereof from the cell debris, e.g. by means of centrifugation; preferably removing DNA's from the crude extract, e.g. by treatment of the crude extract with a solution of strepomycin sulphate; fractionating the crude extract in order to secure fractions containing the restriction enzyme in accordance with the present invention; and isolating the restriction enzyme in accordance with the present invention from the fractions containing it by one or more chromatographic separation steps, e.g. ion exchange chromatography, wherein phosphocellulose or DEAE-cellulose is employed, and/or affinity chromatography, wherein heparin-agarose or DNA-cellulose is employed, and/or gel filtration, wherein heparine-sepharose is employed.

In order that the present invention, together with its various features and advantages, can be more readily understood it will now be further described, though only by way of illustration, in conjunction with the accompanying drawings, in which:

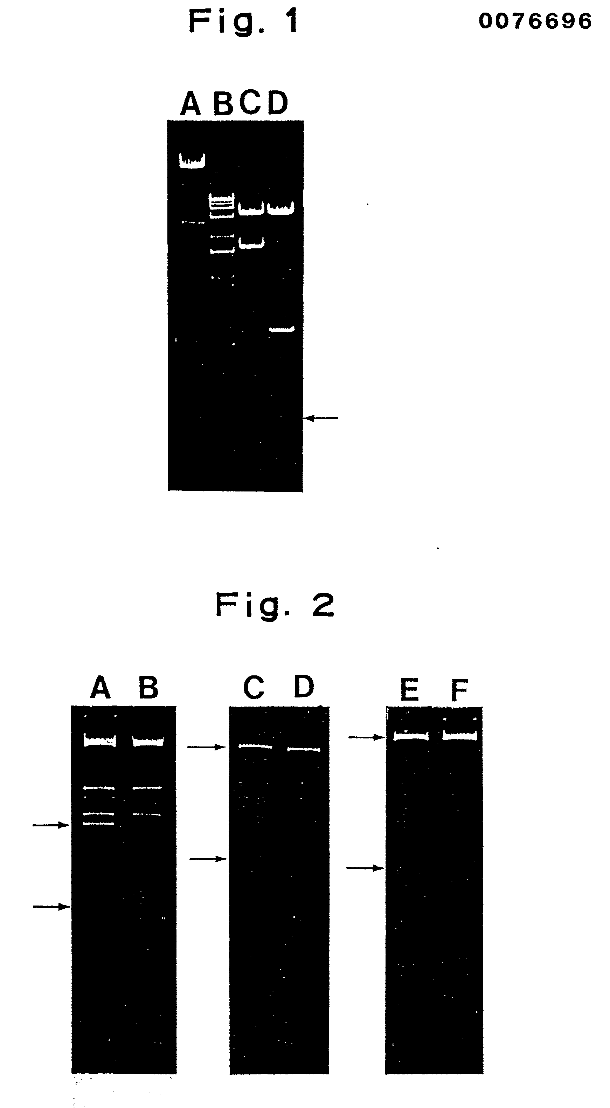

Fig. 1 is a reproduction of a photograph showing resultant band patterns of electrophoresis applied to the digests which were produced by digesting (A) λDNA, (B) Ad2 DNA, (C) ⌀X174 RF DNA, and (D) pBR322 DNA with BbeI, the restriction enzyme in accordance with the present invention;

Fig. 2 is a reproduction of a photograph showing resultant band patterns of electrophoresis applied to the digests which were produced by digesting pBR322 with (A) TaqI, (B) a mixture of TaqI and BbeI, the restriction enzyme in accordance with the present invention, (C) HinfI, (D) a mixture of HinfI and BbeI, the restriction enzyme in accordance with the present invention, (E) a mixture of BamHI and SalI, and (F) a mixture of BamHI, SalI and BbeI, the restriction enzyme in accordance with the present invention;

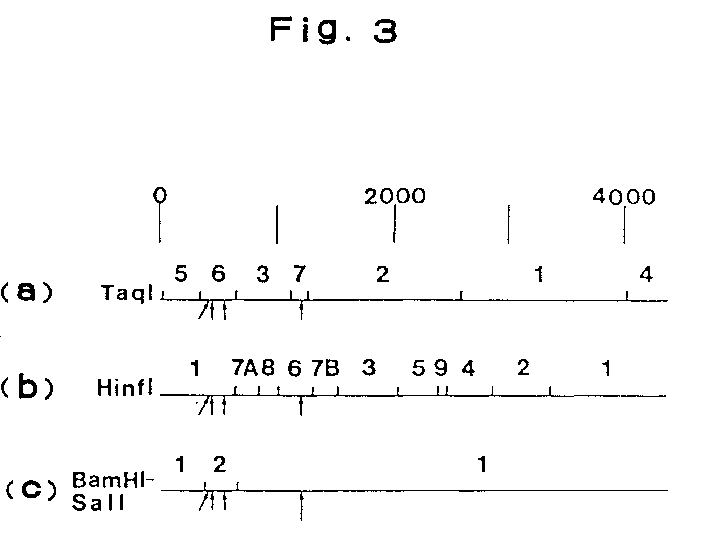

Fig. 3 is a set of three pictures each of which respectively compares (a) the cleavage sites at which pBR322 was cleaved by TaqI and which are shown by notches indicated at locations measured by the number of bp's counted beginning at the unique PstI site and the assumed (and finally determined) cleavage sites at which BbeI cleaves pBR323 and which are shown by arrows indicated at locations measured by the number of bp's counted beginning at the unique PstI site, (b) the cleavage sites at which pBR322 was cleaved by HinfI and which are shown employing the same scale as was employed above and the cleavage sites of BbeI identical to the above, (c) the cleavage sites at which pBR322 was cleaved by a mixture of BamHI and SalI and which are shown employing the same scale as was employed above and the cleavage sites of BbeI identical to the
above, and each of which pictures is accompanied by the
numerals showing the numbers given to each fragment in the
order of length of the fragments, and

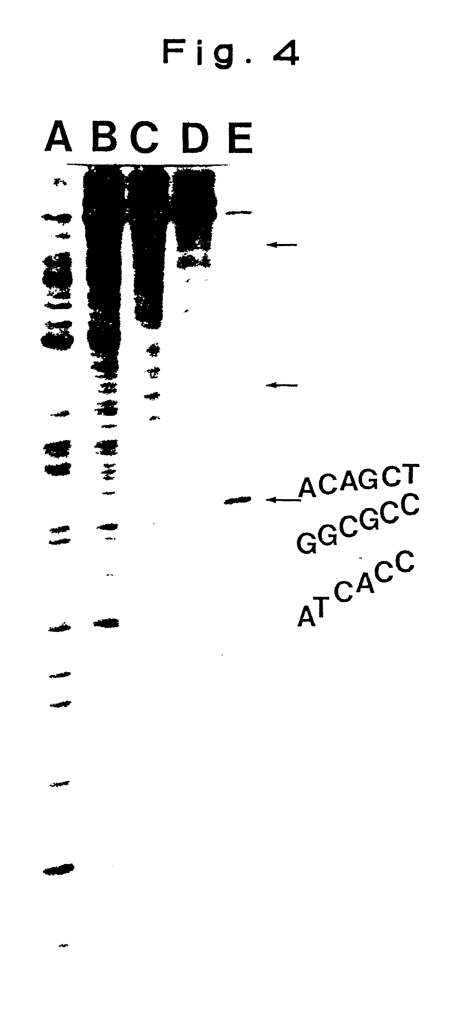

Fig. 4 is a set of reproduction of photographs
showing resultant band patterns of electrophoresis applied
to (A) the fragments having G ends and which are isolated,
by means of a chemical modification procedure, from the
group of the smaller fragments produced by cleaving pBR322
with BamHI and SalI, (B) the fragments having G ends or A
ends and which are isolated, by means of a chemical modification procedure, from the group of the smaller fragments
produced by cleaving pBR322 with BamHI and SalI, (C) the
fragments having T ends or C ends and which are isolated,
by means of a chemical modification procedure, from the
group of the smaller fragments produced by cleaving pBR322
with BamHI and SalI, (D) the fragments having C ends and
which are isolated, by means of a chemical modification
procedure, from the group of the smaller fragments produced
by cleaving pBR322 with BamHI and SalI, and (E) the fragments
produced by cleaving the smaller fragments produced by
cleaving pBR322 with BamHI and SalI, with BbeI, the restriction enzyme in accordance with the present invention, so that
the positions shown on column (E) of the fragments produced
by cleaving the smaller fragments produced by cleaving
pBR322 with BamHI and SalI, with BbeI are compared with the
corresponding positions shown on the other columns, (A), (B), (C) and (D), so as thus to determine the cleavage site of BbeI, the restriction enzyme in accordance with the present invention.

The advance achieved in the development of a restriction enzyme in accordance with the present invention and a method for production of the restriction enzyme in accordance with the present invention will be described below, thus demonstrating the successful creation of the foregoing restriction enzyme, and indeed also the specificity of the foregoing restriction enzyme will be adequately established in accordance with the methodology conventional in the prior art. Further, the completion of an invention directed to the foregoing method for production of the restriction enzyme will be clarified.

Aiming at diversity of restriction enzymes or of the specificity of restriction enzymes, and aiming at development of efficient methods for production of restriction enzymes, we have made research efforts employing some of the intestinal bacteria including the strains itemized above as potential productive strains; and we have succeeded in discovering a restriction enzyme in accordance with the present invention and in elaborating a method for production of the restriction enzyme in accordance with the present invention, when we employed a strain belonging to Bifidobacterium breve, specifically YIT 4006 having its Deposition No, FERM-P3906. The restriction enzyme thus developed has been named BbeI.

## (A) CULTIVATION AND HARVESTING OF STRAIN

The laboratory strain YIT 4006 having its Deposition No. FERM-P3906 was originally isolated from human faeces and maintained on stab in a modified VL-G medium, which contains per liter 7.5 ml of salt solution I (3 % $K_2HPO_4$), 7.5 ml of salt solution II (a mixture of 3 % $KH_2PO_4$, 3 % $(NH_4)_2SO_4$, 6 % NaCl, 0.3 % $MgSO_4 \cdot 7H_2O$ and 0.3 % $CaCl_2 \cdot 2H_2O$), 1 ml of 0.1 % resazulin, 10 gr of Trypticase, 5 gr of yeast extract, 2 gr of meat extract, 10 gr of glucose, 50 ml of 8 % $Na_2CO_3$, 10 ml of 0.07 % hemin, and 10 ml of 3 % cysteine·HCl. Since the cultivation of the laboratory strain could be carried out in conventional manner, the process of cultivation is not limited.

Cells were grown anaerobically at 37°C in the foregoing modified VL-G medium to the early stationary phase. The cells were harvested by centrifugation, and stored at -20°C until use. The determined yield was approximately 4-6 gr of cells per liter of medium.

## (B) PURIFICATION OF BbeI

Frozen cell pellets (60 gr) were thawed and suspended in 240 ml of buffer A (10 mM potassium phosphate, pH 7.0, 7mM 2-mercaptoethanol and 1 mM EDTA) containing 0.1 mM phenylmethylsulfonylfluoride and 0.4 M NaCl.

The cell suspension was treated on ice with lysozyme (100 $\mu$ gr/ml) for 30 min to make the cells susceptible to sonication. Sonication was carried out until more than 90 % of the cells were disrupted.  Care was taken to keep the temperature below 10°C.  All the subsequent operations were carried out at 0-5°C.

The sonicated cell suspension was centrifuged at 100,000 x G for 2 h at 2°C and the supernatant was decanted to remove the cell debris.

A 10 % (w/v) solution of streptomycin sulphate was slowly added to the supernatant until the concentration of streptomycin reaches 1.2 % in the supernatant.  After stirring for additional 30 min, the precipitate in which the major portion of the DNA was contained was removed by slow speed centrifugation.

The supernatant from which the DNA was removed was fractionated with ammonium sulphate.  Each cut acquired by the foregoing fractionation was dissolved in buffer A containing 0.1 M NaCl and was dialyzed overnight against the same buffer.

Each cut was assayed for its specific endonuclease activity.  20 $\mu$l of each cut was added to 30 $\mu$l of a reaction mixture containing 10 mM Tris-HCL, pH 7.4, 8 mM $MgCl_2$, 7 mM 2-mercaptoethanol, and 1 $\mu$gr of E. coli plasmid pBR322.  After allowing  reaction   at 37°C for one h, agarose gel electrophoresis was applied to each cut. Endonuclease activity was positively determined for a cut which could digest the entire quantity of 1 $\mu$gr of pBR322 DNA.  Results of the agarose gel electrophoresis showed endonuclease activity specific for BbeI for 40-60 % cut.

DNA topoisomerase activity was found exclusively in 0-40 % cut.

The foregoing 40-60 % cut was further purified by combination of gel filtration, ion exchange and affinity chromatographies.

More specifically, the foregoing 40-60 % cut was applied to a column (1.5 x 32 cm) of phosphocellulose (p 11, Whatman) which was previously equilibrated with buffer A containing 0.1 M NaCl. After the column was washed with 120 ml of the same buffer, it was developed with 400 ml of buffer A containing 0.1-0.6 M NaCl to elute the endonuclease activity in accordance with the present invention. Enzyme assay which is similar to that which was previously employed was applied to each of the eluted fractions to determine the endonuclease activity thereof. The results of the test showed endonuclease activity specific for BbeI for the fractions eluted with 0.29-0.34 M NaCl.

All of the fractions eluted with 0.29-0.34 M NaCl were pooled and dialyzed against buffer B (10 mM Tris-HCL, pH 7.4, 7 mM 2-mercaptoethanol and 1 mM EDTA). The dialyzed sample was loaded on a DEAE-cellulose (DE52, Whatman) column (1.0 x 20 cm) which had been equilibrated with buffer B. After washing with 35 ml of buffer B, the column was developed with 160 ml of buffer B containing 0-0.5 M NaCl to elute BbeI. Enzyme assay, which is similar to that which was previously employed, was applied to each of the eluted fractions to determine the endonuclease activity of each fraction.  The results of the test showed endonuclease activity specific for BbeI for the fractions eluted  with 0.21-0.26 M NaCl.

All of the fractions eluted  with 0.21-0.26 M NaCl were pooled and adjusted to 0.25 M NaCl, before being applied to heparin-Sepharose CL-6B (Pharmacia) column (1.0 x 8.5 cm) which had been equilibrated with buffer B containing 0.25 M NaCl.  The column was extensively washed and developed with 70 ml of buffer B containing 0.25-0.75 M NaCl.  Enzyme assay,which is similar to that which was previously employed,was applied to each fraction to determine the endonuclease activity of each fraction.  The results of the test showed endonuclease activity specific for BbeI for fractions eluted  with 0.33-0.41 M NaCl.

All of the fractions eluted  with 0.33-0.41 M NaCl were co-mingled and dialyzed against buffer B.  This preparation was further purified on double stranded DNA-cellulose column.  The dialyzed sample was applied to the column ( 1.0 x 5 cm) which had been equilibrated with buffer B.  After being applied with extensive washing, the sample was developed with 50 ml of buffer B containing 0-0.5 M NaCl.  The results of the test showed endonuclease activity specific for BbeI for fractions eluated with 0.07-0.16 M NaCl.

All of the fractions eluted  with 0.07-0.16 M NaCl were pooled, concentrated by dialysis against buffer B containing 50 % glycerol, and added bovine serum albumin to a concentration of 500 $\mu$gr/ml.

pBR322 was digested by an excess quantity of this
BbeI preparation, before the digested sample was applied with
gel electrophoresis.  The electrophoresis gave sharp bands
to show that the BbeI preparation is essentially free of
nonspecific nucleases.  Further, enzyme assay carried out
on non-substrate SV40 DNA showed that this BbeI preparation
contained no detectable activity of DNA topoisomerase.  As
a result, we determined that the above process has success-
fully isolated BbeI.

The BbeI was stored at -20°C for more than a year
without significant loss of activity.

(C) RECOGNITION SEQUENCE OF BbeI

Referring to Fig. 1, BbeI was employed to digest
various DNA's of which the sequences were known in 10 mM
Tris·HCl, pH 7.4, 8 mM $MgCl_2$, 7 mM 2-mercaptoethanol.
Electrophoresis on a 1.4 % agarose gel plate was applied to
the digested products.  The results are shown in Fig. 1.
As seen in columns A, B, C and D in Fig. 1, respectively,
it is clear that BbeI cleaved λDNA, Ad2 DNA, ⌀X174 RF DNA
and pBR322 DNA respectively into 2, 18, 2, and 3.  The arrow
to the right of column D indicates the third largest fragment
of the digested product of pBR322 DNA.  Incidentally, BbeI
did not cleave SV40 DNA nor fd RF DNA.  Referring to the
table tabulating the sequencing data for sequenced DNA's
prepared and published by Fuchs et al., we assumed that
BbeI is capable of recognizing a palindromic hexanucleotide
sequence 5'-GGCGCC-3'.

Referring to Fig. 2, the BbeI cleavage sites on pBR322 DNA were analyzed. pBR322 DNA was digested with (A) TaqI, (B) a mixture of TaqI and BbeI, (C) JinfI, (D) a mixture of HinfI and BbeI, (E) a mixture of BamHI and SalI, and (F) a mixture of BamHI, SalI and BbeI. The digested products were analyzed by electrophoresis on a 5 % polyacrylamide gel in 40 mM Tris, 20 mM sodium acetate, 2 mM EDTA, pH 7.8. The results are shown in Fig. 2. Arrows indicate the bands which were cleaved by BbeI. As seen in columns A and B of Fig. 2, BbeI cleaved the smallest fragments 6 and 7 generated by TaqI. As seen in columns C and D of Fig. 2, BbeI cleaved the fragments 1 and 6 generated by HinfI. In addition, as seen in columns E and F of Fig. 2, the smaller fragment which is 275 bp long and which is flanked by BamHI and SalI sites was cleaved by BbeI.

Referring to Fig. 3, all the BbeI cleavage sites were found to be located in coordinate nucleotide sequences 375-631 and 1125-1266 on the DNA sequence of Sutcliffe (1978).

By scanning these two regions for common sequences it was found that only a palindromic hexanucleotide sequence 5'-GGCGCC-3' occurs at coordinate nucleotide positions 416, 437, 550 and 1207. Accordingly, we concluded that BbeI, the restriction enzyme in accordance with the present invention, is capable of recognizing a palindromic hexanucleotide sequence 5'-GGCGCC-3'.

(D) CLEAVAGE SITE OF BbeI

The method of Maxam and Gilbert was employed to determine the cleavage site of BbeI.

Referring to Fig. 4, pBR322 DNA was cleaved to linear molecules with BamHI, and dephosphorylated with bacterial alkaline phosphatase, before the cleaved fragments are labeled at both 5' ends with $(\gamma-^{32}P)$ ATP and T4 polynucleotide kinase. The labelled molecules were digested with SalI. Of the thus-produced fragments, the smaller fragment having 275 bp was isolated by electrophoresis on a 7 % polyacrylamide gel plate. An aliquot of this fragment containing three BbeI sites at coordinate nucleotide positions 416, 437 and 550 was partially digested with BbeI. It was subjected to electrophoresis on a 15 % polyacrylamide thin slab gel in a column adjacent to a sequence ladder prepared from the remaining aliquot of the labeled fragment with a chemical modification procedure. More specifically, out of the fragments produced by the foregoing double digestion processes, the group of the smaller fragments were isolated from the others. Out of the smaller fragments, (A) the fragments having G ends, (B) the fragments having G ends or A ends, (C) the fragments having T ends or C ends, and (D) the fragments having C ends were respectively isolated, by means of a chemical modification procedure, from the others, before being applied with electrophoresis, of which the resultant band patterns were shown respectively in columns (A), (B), (C), and (D) of Fig. 4. Incidentally, the smaller fragments were digested with BbeI, and electrophoresis was applied to the produced fragments.  Column (E) of Fig. 4
shows the resultant band pattern of the electrophoresis.

Three groups of the labelled fragments produced by
digestion of the foregoing smaller fragments with BbeI were
seen in column (E) of Fig. 4, as indicated by the arrows.
The second fragment from the bottom could be determined to
be exactly 21 bp longer than the fastest-moving fragment as
expected from the DNA sequence.

From the observation of the positions of the BbeI
cleavage fragments shown by arrows arranged beside column
(E) of Fig. 4, in comparison with the band patterns shown
in columns (A), (B), (C) and (D) of Fig. 4, it was possible
to determine that the cleavage site of BbeI, the restriction
enzyme in accordance with the present invention, lies
between the two C's at the 3' end of its palindromic recognition sequence 5'-GGCGCC-3'.  Incidentally, it was possible
to determine that BbeI, the restriction enzyme in accordance
with the present invention, generates DNA fragments with
tetranucleotide long 3'-prodruding ends, GCGC-3'.

The foregoing description has demonstrated that a
restriction enzyme specified above was successfully discovered
or created, the specificity thereof was successfully estab
lished , and a method for production thereof was successfully
developed.

Albeit the present invention has been described with
reference to a specific strain, YIT 4006 having its Deposition
No. FERM-P3906, this description is not meant to be construed

**0076696**

in a limiting sense. Various modifications of the described embodiments, as well as other embodiments of this invention, will become apparent to persons skilled in the art upon reference to the description of the present invention. It is therefore contemplated that the apended claims will cover any such modifications or embodiments as fall within the true scope of this invention.

**CLAIMS**

1. A restriction enzyme having a specificity such that it can recognize a palindromic hexanucleotide sequence

5'-GGCGCC-3'
3'-CCGCGG-5',

in double-stranded deoxyribonucleic acids, and can cleave the phosphodiester bonds contained in said double-stranded deoxyribonucleic acids between the two C's at the 3' ends shown by arrows on the following sequence,

5'-GGCGCC-3'
3'-CCGCGG-5',

so that deoxyribonucleic acid fragments having tetranucleotide long 3'-protruding ends are generated.

2. A restriction enzyme having a specificity such that it can recognize a palindromic hexanucleotide sequence

5'-GGCGCC-3'
3'-CCGCGG-5'

in double-stranded deoxyribonucleic acids, and can cleave the phosphodiester bonds contained in said double-stranded deoxyribonucleic acids between the two C's at the 3' ends shown by arrows on the following sequence

5'-GGCGCC-3'
3'-CCGCGG-5'

so that deoxyribonucleic acid fragments having tetranucleotide long 3'-protruding ends are generated, and also having an optimum pH value of 7.4 within a stable pH range of from 6.5 to 8.3, and an optimum temperature range of from $35^\circ$ to $39^\circ$C.

3.   A method of producing a restriction enzyme, which comprises the steps of cultivating a strain of <u>Bifidobacterium breve</u> and extracting the desired restriction enzyme from the culture thus produced.

4.   A method for production of a restriction enzyme which comprises the steps of cultivating a strain belonging to <u>Bifidobacterium breve</u>;

harvesting cells of said strain;

disrupting said cells;

separating a crude extract of said cells;

fractionating said crude extract; and

purifying said crude extract at least once so as thus to isolate therefrom a restriction enzyme having specificity for recognizing a palindromic hexanucleotide sequence

        5'-GGCGCC-3'
        3'-CCGCGG-5',

in double-stranded deoxyribonucleic acids, and for cleaving said deoxyribonucleic acids between the two C's at the 3' ends.

5.   A method for production of a restriction enzyme as claimed in claim 4, in which the strain is the specific strain of which the Deposition NO. is FERM-P3906.

6.   A restriction enzyme whenever produced by the method claimed in any of claims 3 to 5.

7.   A method of cleaving a deozyribonucleic acid by contacting it with a restriction enzyme, in which method the restriction enzyme employed is that claimed in claim 1, claim 2 or claim 6.

8.    The cleavage products of deoxyribonucleic acid whenever obtained by the method of claim 7.

# Fig. 1

# Fig. 2

# Fig. 3

0076696

# Fig. 4

A B C D E

ACAGCT

GGCGCC

ATCACC